# EUROPEAN PATENT SPECIFICATION

(11) **EP 2 866 774 B1**
(45) Date of publication and mention of the grant of the patent: **09.08.2017**
(21) Application number: 12735068.4
(22) Date of filing: 29.06.2012
(51) Int. Cl.: A61K 8/25, A61K 8/73, A61Q 11/00

(54) **HIGH WATER CONTENT ORAL COMPOSITIONS COMPRISING MICROCRYSTALLINE CELLULOSE AND CARBOXYMETHYLCELLULOSE**
ORALE ZUSAMMENSETZUNGEN MIT HOHEM WASSERGEHALT MIT MIKROKRISTALLINER CELLULOSE UND CARBOXYMETHYLCELLULOSE
COMPOSITIONS ORALES À TENEUR EN EAU ÉLEVÉE COMPRENANT DE LA CELLULOSE MICROCRISTALLINE ET DE LA CARBOXYMÉTHYLCELLULOSE

(43) Date of publication of application: 06.05.2015
(73) Proprietor: Colgate-Palmolive Company, New York, NY 10022 (US)
(72) Inventor: MELLO, Sarita, North Brunswick, New Jersey 08902 (US); PRENCIPE, Michael, West Windsor, New Jersey 08550 (US); FISHER, Steve, Middlesex, New Jersey 08846 (US); LAMBERT, Pierre, B-4621 Retinne (Fleron) (BE); DELVENNE, Jean-Paul, B-4100 Seraing (BE); VOGT, Robert, Princeton Junction, New Jersey 08550 (US)
(74) Representative: Wibbelmann, Jobst
(86) International application number: PCT/US2012/044832
(87) International publication number: WO 2014/003776

(56) References cited:
- EP-A1- 2 100 590
- EP-A2- 2 371 348
- WO-A2-2004/071321
- US-A- 5 601 803

## Description

### BACKGROUND

High-water toothpastes are desirable, as they can reduce the amount of ingredients, such as custom silica abrasives, but they are technically challenging. When the level of water in a formulation is higher than 20%, the flavor delivery and rheological profile may become unacceptable, causing comments like: "watery formula, flavor not fresh enough, flavor does not last long", etc. Silica abrasive dentifrices present particular challenges in that the silica interacts with water to provide bulk and body to the toothpaste, and the flavor components may adsorb or interact with the silica. Thickening systems to enhance the viscosity at higher water levels are known, but may interfere with the flavor, and may not provide a desirable rheology, especially after having been extruded from the toothpaste tube or other container. Changing the water concentration changes the flavor/surfactant micelle, which is ultimately linked to the interaction of flavor with silica and polymeric ingredients, such as natural/synthetic cellulose and carrageenans, and humectant agents, as well as changing the rheological profile of the dentifrice.

Cellulose thickeners, such as mixtures of microcrystaliine cellulose and carboxymethyl cellulose, are generally inexpensive and have been used in toothpaste formulations, e.g., as disclosed in our US Patent 5,601,803. The formulations disclosed therein were relatively simple, however, comprising a single abrasive silica system and a sorbitol/glycerin/polyethylene glycol/water humectant system, with a relatively low water content. Certain ultra-high water (>50% water) dentifrice formulations comprising microcrystaliine cellulose are described in our co-pending application PCT/US11/66093, filed December 20, 2011.

There is a need for high water toothpaste formulations that are both cost effective and maintain desirable rheology and flavor delivery.

EP2371348 describes an oral care composition.

EP2100590 describes an oral composition containing crystalline cellulose surface-treated with water-soluble substance.

WO2004/071321 describes toothpaste compositions with reduced abrasivity.

### SUMMARY

We have found that incorporating microcrystalline cellulose into high-water dentifrice formulations greatly improves the flavor delivery and rheology profile as compared to a control toothpaste without microcrystalline cellulose.

The invention therefore provides, in a first embodiment, dentifrice compositions (Composition 1) comprising by weight
a. 35-45% of water;
b. 0.7% to 1.2% of a component for enhancing flavor delivery and rheological profile, wherein the component comprises a blend of:
   b1. 80% to 90% of a microcrystalline cellulose; and
   b2. 10% to 20% of sodium carboxymethylcellulose;
   wherein the weight percentage of b1. and b2. is based on the total weight of component; and
c. an effective amount of a silica abrasive, wherein the silica abrasive component comprises 15-25% of the composition.
wherein the composition is obtained or obtainable by premixing the blend of microcrystalline cellulose and sodium carboxymethyl cellulose in water prior to combination with the other ingredients.

Composition 1 may further comprise, e.g., surfactants, foaming agents, vitamins, polymers, enzymes, humectants, thickeners, antimicrobial agents, preservatives, flavorings, colorings and/or combinations thereof.

The properties of such compositions are improved when the microcrystalline cellulose is first dispersed in water before the other ingredients are added. Accordingly, the invention further provides a method of making a dentifrice composition of Composition 1, comprising dispersing the blend of 80-90% microcrystalline cellulose and 10-20% sodium carboxymethyl cellulose in water prior to addition of the other ingredients, wherein the blend of 80-90% microcrystalline cellulose and 10-20% sodium carboxymethyl cellulose is in an amount corresponding to 0.7-1.2%) of the final product.

The invention further provides compositions of the invention, e.g., Composition 1, for use in the following: to clean the teeth, reduce plaque, reduce gingivitis, inhibit tooth decay and formation of cavities, and reduce dentinal hypersensitivity. In some embodiments, the use comprises brushing the teeth with Composition 1.

Further areas of applicability of the present invention will become apparent from the detailed description provided hereinafter.

### DETAILED DESCRIPTION

The following description of the preferred embodiment(s) is merely exemplary in nature and is in no way intended to limit the invention, its application, or uses.

The invention therefore provides, in a first embodiment, a dentifrice composition (Composition 1) comprising by weight
a. 35-45% of water;
b. 0.7% to 1.2% of a component for enhancing flavor delivery and rheological profile, wherein the component comprises a blend of:
   b1. 80% to 90% of a microcrystalline cellulose; and
   b2. 10% to 20% of sodium carboxymethylcellulose;
   wherein the weight percentage of b1. and b2. is based on the total weight of component; and
c. an effective amount of a silica abrasive, wherein the silica abrasive component comprises 15-25% of the composition,
wherein the composition is obtained or obtainable by premixing the blend of microcrystalline cellulose and sodium carboxymethyl cellulose in water prior to combination with the other ingredients.

Embodiment of the invention include, but are not limited to:
1.1. Composition 1 further comprising a synthetic anionic polymeric polycarboxylate.
1.2. The foregoing composition wherein the anionic polymer is a 1:4 to 4:1 copolymers of maleic anhydride or acid with another polymerizable ethylenically unsaturated monomer.
1.3. The foregoing composition wherein the anionic polymer is a methyl vinyl ether/maleic anhydride (PVM/MA) copolymer having an average molecular weight (M.W.) of about 30,000 to about 1,000,000, e.g. about 300,000 to about 800,000.
1.4. Any of the foregoing compositions wherein the anionic polymer is about 1-5, e.g., about 2% of the weight of the composition.
1.5. Any of the foregoing compositions wherein the silica abrasive component comprises (a) a first population of silica abrasive particles, having a d50 of less than 5 microns, e.g., 3-5 microns, e.g. about 4 microns, e.g. 3.95 microns, and (b) a second population of silica abrasive particles, having a d50 of greater than 8 microns, e.g. 8-13 microns, wherein the ratio of the first population to the second population by weight is 1:2 to 1:4, e.g. about 1:3.
1.6. Any of the foregoing compositions further comprising an effective amount of fluoride, e.g., wherein the fluoride is a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.
1.7. Any of the foregoing compositions comprising L-arginine in free or orally acceptable salt form, e.g in an effective amount e.g. to reduce dentinal hypersensitivity and/or plaque accumulation, e.g. 1-10%.
1.8. Any of the foregoing compositions comprising buffering agents, e.g., sodium phosphate buffer (e.g., sodium phosphate monobasic and disodium phosphate). 1.9.
Any of the foregoing compositions comprising a humectant, e.g., selected from glycerin, sorbitol, propylene glycol, polyethylene glycol, xylitol, and mixtures thereof, e.g. comprising at least 20%, e.g., 20-40%, e.g., 25-35% glycerin.
1.10. Any of the preceding compositions comprising one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS).
1.11. Any of the preceding compositions further comprising a viscosity modifying amount of one or more of polysaccharide gums, for example xanthan gum or carrageenan, silica thickener, and combinations thereof.
1.12. Any of the preceding compositions comprising gum strips or fragments.
1.13. Any of the preceding compositions further comprising flavoring, fragrance and/or coloring.
1.14. Any of the foregoing compositions comprising an effective amount of one or more antibacterial agents, for example comprising an antibacterial agent selected from natural extracts which include, but are not limited to, those isolated from green or oolong tea, oregano. gold thread, cranberry and other Ericaceae family plants, honeysuckle, grape seed, myrobalan, rosemary, east Indian walnut, neem, niruri, and pine bark. Other natural extracts that are known antimicrobial agents are those listed in the International Cosmetic Ingredient Dictionary and Handbook, Tenth Ed., 2004, including the following extracts. Grape seed is extracted from Vitis Vinifera seed. Myrobalan is preferably extracted from Terminalia Bellerica fruit. Rosmarinus Officinalis leaf is isolated to create rosemary extract. Pine bark extract is preferably extracted from the cortex (bark) of Pinus Pinaster (Maritime pine). The leaf of East Indian walnut (Albizia Lebbek) is used for the extract. The extract of the cortex of the neem or margosa plant (Melia Azadirachta) is a known antibacterial component. Niruri or Phyllanthus Niruri extract is also a known antibacterial extract. Also included as antibacterial agents are magnolol, tetrahydromagnolol, butyl magnolol, honokiol and tetrahydrohonokiol. Extracts suitable for use in the present invention can be obtained from any part of the plant including the leaf, stem, bark, pulp, seed, flesh, juice, root and mixtures thereof. It is preferred that the extract is obtained from the leaf, pulp and seed, more preferably from the leaf, flower or bark. The natural extracts containing antibacterial active compounds that are useful as additional antiplaque, e.g., antibacterial agents, in the oral compositions should be safe and suitable for use in mammals.
1.15. Any of the foregoing compositions comprising an effective amount of one or more antibacterial agents which also include non-ionic and anionic agents known to one of skill in the art. Examples of non-ionic agents include substantially water insoluble, noncationic antibacterial agents. For example, such antibacterial agents include an alkylphenoxy phenol; a cycloalkyl-phenoxyphenol; a 9,10-dihydrophenanthrenol; an alkylphenol; a cycloalkyl-phenol; a phenolic compound; a halogenated carbanilide; a halogenated salicylanilide; a benzoic ester; a halogenated diphenyl ether, and mixtures thereof. The nonionic antibacterial alkylphenoxy phenol or cycloalkyl-phenoxyphenol or -9,10-dihydrophenanthrenol includes a noncationic antibacterial phenol containing, relative to the hydroxyl group, an alkyl or cycloalkyl group, preferably tert-butyl (t-butyl), in 2-position, and substituents in one or both of the 4- and 5-positions, one of which may be phenyl or 2', 3' and/or 4' substituted alkyl or cycloalkyl phenyl, preferably 4'-t-butyl phenyl or a phenanthrene containing a hydroxyl substituent in the 2- or 3-position and alkyl or cycloalkyl, preferably t-butyl, substituents in the other of the 2- and 3-positions and in at least one of the other rings and are described in U.S. Pat. No. 5,723,500 to Stringer et al., issued Mar. 3, 1998. The water insoluble non-ionic antibacterial alkyl-phenol or cycloalkyl-phenol include a phenol containing, relative to the hydroxyl group, an alkyl or cycloalkyl group, preferably tert-butyl (t-butyl), in the 2-position, and substituents in one or both of the 4- and 5-positions, one or both of which may be alkyl or cycloalkyl, one being preferably t-butyl, such as those described in U.S. Pat. No. 5,912,274, Stringer et al., Jun. 15, 1999. The phenolic compounds among those useful herein include phenol and its homologs, mono and polyalkyl and aromatic halophenols, resorcinol and its derivatives, and bisphenolic compounds, such as those disclosed in U.S. Pat. No. 5,368,844, Gaffar et al., issued Nov. 29, 1994. Certain preferred phenolic compounds are n-hexyl resorcinol and 2,2'-methylene bis (4-chloro-6-bromophenol). Exemplary halogenated carbanilides, halogenated salicylanilides and benzoic esters are disclosed in U.S. Pat. No. 5,776,435, Gaffar et al., issued Jul. 7, 1998. Halogenated carbanilides include 3,4,4'-trichlorocarbanilide, 3-trifluoromethyl-4,4'-dichlorocarbanilide, and 3,3',4-trichlorocarbanilide. Halogenated salicylanilides include 4'5-dibromosalicylanilide, 3,4',5-trichlorosalcylanilide, 3,4',5-tribromosalicylanilide, 2,3,3',5-tetrachlorosalicylanilide, 3,3',5-tetrachlorosalicylanilide, 3,5-dibromo-3'-trifluoromethyl salicylanilide, 5-n-octanoyl-3'-trifluoromethyl salicylanilide, 3,5-dibromo-4'-trifluoromethyl salicylanilide, 3,5-dibromo-3'-trifluoro methyl salicylanilide (Fluorophene), and mixtures thereof. Benzoic esters include methyl-p-hydroxybenzoic ester, ethyl-p-hydroxybenzoic ester, propyl-p-hydroxybenzoic ester, and butyl-p-hydroxybenzoic ester. A particularly suitable non-ionic antiplaque antibacterial agent is a diphenyl ether selected from the group comprising 2,4,4'-trichloro-2'-hydroxydiphenyl ether (triclosan) and 2,2'-dihydroxy-5,5'-dibromodiphenyl ether. Triclosan is particularly suitable for use as an additional antibacterial agent.
1.16. Any of the foregoing compositions comprising an effective amount of one or more antibacterial agents comprising a compound of Formula (I) 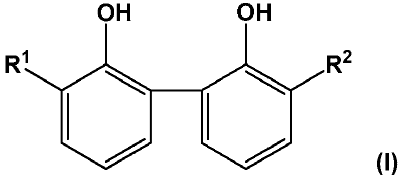 or a salt thereof, wherein R¹ and R² are each independently (C₁-C₆) alkyl or (C₂-C₆) alkenyl, and a carrier. In certain embodiments, the composition is an oral care composition that comprises an antibacterially-effective amount of a compound of Formula (I), or a salt thereof, and an orally-acceptable carrier. In other embodiments, the composition comprises an antibacterially-effective amount of a compound of Formula (I) or a salt thereof, and an dermatologically-acceptable carrier. In certain embodiments, R¹ and R² are independently selected from methyl, ethyl, *n*-propyl, *i*-propyl, *n*-butyl, *i*-butyl, *t*-butyl, 2-methyl-butyl, - *n*-pentyl, *i*-pentyl, *t*-pentyl and hexyl. In other embodiments, R¹ and R² are the same and both are (C₁-C₆) alkyl or (C₂-C₆) alkenyl. In a particular embodiment, R¹ and R² are both *n*-propyl, and the compound of Formula (I) is compound (3), which has the following structure: 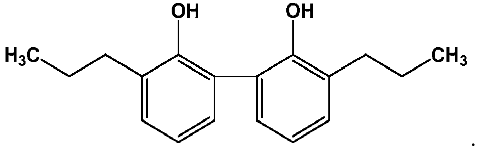
1.17. In various embodiments, the additional antiplaque antibacterial agents added to the oral composition of the present invention comprise about 0.0001% to about 10%, preferably about 0.001% to about 5%, more preferably about 0.01% to about 3%, depending on the concentration of the active compounds and the form of the dentifrice composition.
1.18. Any of the foregoing compositions comprising an antibacterially effective amount of triclosan, e.g. 0.1 -0.5%, e.g. about 0.3%.
1.19. Any of the preceding compositions further comprising a whitening agent, e.g., a selected from the group consisting of peroxides, metal chlorites, perborates, percarbonates, peroxyacids, hypochlorites, and combinations thereof.
1.20. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate);
1.21. Any of the preceding compositions further comprising an agent that interferes with or prevents bacterial attachment, e.g., solbrol or chitosan.
1.22. Any of the preceding compositions further comprising a source of calcium and phosphate selected from (i) calcium-glass complexes, e.g., calcium sodium phosphosilicates, and (ii) calcium-protein complexes, e.g., casein phosphopeptide-amorphous calcium phosphate
1.23. Any of the preceding compositions further comprising a soluble calcium salt, e.g., selected from calcium sulfate, calcium chloride, calcium nitrate, calcium acetate, calcium lactate, and combinations thereof.
1.24. Any of the preceding compositions further comprising a physiologically or orally acceptable potassium salt, e.g., potassium nitrate or potassium chloride, in an amount effective to reduce dentinal sensitivity.
1.25. Any of the preceding compositions further comprising a breath freshener, fragrance or flavoring.
1.26. Any of the preceding compositions effective upon application to the oral cavity, e.g., with brushing, to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; and/or (xv) promote systemic health, including cardiovascular health, e.g., by reducing potential for systemic infection via the oral tissues.
1.27. A composition obtained or obtainable by combining the ingredients as set forth in any of the preceding compositions.
1.28. The compositions of the invention are obtained or obtainable by premixing a blend of 80-90% microcrystalline cellulose and 10-20% sodium carboxymethyl cellulose in water prior to combination with the other ingredients. In one embodiment, the compositions of the invention are obtained or obtainable by dispersing a blend of 80-90% microcrystalline cellulose and 10-20% sodium carboxymethyl cellulose in water, then admixing additional sodium carboxymethyl cellulose and other ingredients.The mixture of 80-90% microcrystalline cellulose and 10-20% sodium carboxymethyl cellulose is in an amount corresponding to 0.7-1.2% of the final product.
1.29. Composition 1 further comprising
   e. 25-35% glycerin
   f. 1-3% PVM/MV copolymer
   g. 0.1-0.5% triclosan.

The invention further provides a method of making a dentifrice composition of any of Composition 1, et seq. above, comprising dispersing the blend of microcrystalline cellulose and sodium carboxymethyl cellulose in water prior to addition of the other ingredients, for example dispersing a blend of 80-90% microcrystalline cellulose and 10-20% sodium carboxymethyl cellulose in water, then admixing additional sodium carboxymethyl cellulose and other ingredients of Composition 1. The mixture of 80-90% microcrystalline cellulose and 10-20% sodium carboxymethyl cellulose is in an amount corresponding to 0.7-1.2% of the final product.

The invention further provides methods of using the compositions of the invention, e.g., to (i) reduce hypersensitivity of the teeth, (ii) to reduce plaque accumulation, (iii) reduce of inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiy) whiten teeth; and/or (xv) promote systemic health, including cardiovascular health, e.g., by reducing potential for systemic infection via the oral tissues, comprising applying any of Compositions 1, et seq. as described above to the oral cavity of a person in need thereof, e.g., by brushing the teeth one or more times per day with any of Compositions 1, et seq. The invention further provides Compositions 1, et seq. for use in any of these methods.

The invention further provides the use of microcrystalline cellulose in the manufacture of a dentifrice as described herein, e.g., to reduce the amount of silica abrasive required, or for use in any of the foregoing methods. In an embodiment of the invention, the dentifrice composition contains glycerin in an amount from 20-40% or 25-35%.

*Microcrystalline cellulose:* Microcrystalline cellulose is available from a variety of commercial sources. In one embodiment, the microcrystalline cellulose is provided as a blend of microcrystalline cellulose and sodium carboxymethyl cellulose, for example 80-90% microcrystalline cellulose and 10-20% sodium carboxymethyl cellulose (e.g., Avicel® CL611 from FMC BioPolymer - (about 81.2% - 88.7% microcrystalline cellulose and about 11.3-18.8% sodium carboxymethylcellulose) - product literature for Avicel® CL611 recommends a starting use level of 2.6%; for Avicel® RC591, a use level of 1.2%).

*Active Agents:* The effective concentration of the active ingredients used herein will depend on the particular agent and the delivery system used. It is understood that a toothpaste for example will typically be diluted with water upon use, while a mouth rinse typically will not be. Thus, an effective concentration of active in a toothpaste will ordinarily be 5-15x higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Arginine, where present, may be present at levels from, e.g., about 0.1 to about 20 wt % (expressed as weight of free base), e.g., about 1 to about 10 wt % for a consumer toothpaste or about 7 to about 20 wt % for a professional or prescription treatment product. Fluoride where present may be present at levels of, e.g., about 25 to about 25,000 ppm, for example about 750 to about 2,000 ppm for a consumer toothpaste, or about 2,000 to about 25,000 ppm for a professional or prescription treatment product. Levels of antibacterial agents will vary similarly, with levels used in toothpaste being e.g., about 5 to about 15 times greater than used in mouthrinse. For example, a triclosan toothpaste may contain about 0.3 wt % triclosan.

*Fluoride Ion Source:.* The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counterion in the salt.

*Abrasives:* The compositions of the invention, e.g. Composition 1 et seq. include silica abrasives, and may comprise additional abrasives, e.g., a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof.

Where the silica abrasive component comprises (a) a first population of silica abrasive particles, having a d50 of less than 5 microns, e.g., 3-5 microns, e.g. about 4 microns, e.g. 3.95 microns, and (b) a second population of silica abrasive particles, having a d50 of greater than 8 microns, e.g. 8-13 microns, wherein the ratio of the first population to the second population by weight is 1:2 to 1:4, e.g. about 1:3, the first population may be, e.g., a silica as described in United States Patent Application 2009/0186090 (the contents of which are incorporated herein by reference), e.g., Sorbosil AC43 silica from PQ Corp.; and the second population may be, e.g., a silica as described in United States Patent Application 2012/0100193 (the contents of which are incorporated herein by reference), e.g., Sylodent VP5 from W.R.Grace.

Particle size distribution may be measured using a Malvern Particle Size Analyzer, Model Mastersizer 2000 (or comparable model) (Malvern Instruments, Inc., Southborough, Mass.), wherein a helium-neon gas laser beam is projected through a transparent cell which contains silica, such as, for example, silica hydrogel particles suspended in an aqueous solution. Light rays which strike the particles are scattered through angles which are inversely proportional to the particle size. The photodetector measures the quantity of light at several predetermined angles. Electrical signals proportional to the measured light flux values are then processed by a microcomputer system, against a scatter pattern predicted from theoretical particles as defined by the refractive indices of the sample and aqueous dispersant to determine the particle size distribution of the silica hydrogel, for example. It will be understood that other methods of measuring particle size are known in the art, and based on the disclosure set forth herein, the skilled artisan will understand how to calculate median particle size, mean particle size, and/or particle size distribution of silica particles of the present invention.

The first population of silica abrasive particles having a d50 of less than 5 microns provides particles that have a median particle size that is no greater than the average diameter of a mammalian dentin tubule, such that one or more particles is/are capable of becoming lodged within the tubule, thereby effecting a reduction or elimination of perceived tooth sensitivity. The particles may have a median particle size of about 2 µm to about 4 µm, a d10 of about 0.5 µm to about 2 µm, and a d90 of about 5 µm to about 10 µm. As used herein, d10 refers to particles having a diameter that is 10% of the threshold of the sampled population (i.e., 10% of the population is equal to or smaller than the d10 value), and d90 refers to particles having a diameter that is 90% of the threshold of the sampled population (i.e., 90% of the population is equal to or smaller than the d90 value). In another aspect, a silica has a particle size characterized by a median particle size of about 3 µm to about 5 µm, a d10 of about 1.5 µm to about 3 µm, and a d90 of about 6 µm to about 11 µm. In one embodiment, the first population of silica abrasive particles has a d50 of about 3.95 µm (i.e., 50% of the population of silica particles is equal to or smaller than the d50 value). In one embodiment, the first population of silica abrasive particles has an average particle size of 2.7-4.0 microns (as determined by MALVERN MASTERSIZER), a sieve residue of +45 µm, a moisture loss at 105° C. of 8.0% max, an ignition loss at 1000° C. of 14.0% max, and a pH of 5.5-7.5 in aqueous suspension. In one embodiment, these silica particles have a porosity of less than about 0.45 cc/g in pores of about 600 Angstroms or smaller.

The second population of silica abrasive particles, having a d50 of greater than 8 microns, may in some embodiments break down or fracture as the oral composition is brushed against hard dental surfaces, e.g., dentin or enamel, despite having a relatively low Einlehner hardness value, e.g., 4 to 11, and maintaining their integrity when brushed against softer tissue such as the gums, the mean particle size of the silica compound may be reduced by 10% or more after being applied to a hard surface in an oral cavity. Einlehner hardness may be determined by various means known by those of skill in the art. For example, an Einlehner At-1000 Abrader may measure the hardness of the abrasive particle in the following manner: a Fourdrinier metal screen, i.e., copper or brass, is weighed and exposed to the action of a suspension of the abrasive (for example, a 10% aqueous suspension of the abrasive) for a given number of revolutions. The hardness value is expressed as milligrams weight lost of the Fourdrinier wire screen per number of revolutions, e.g., 100,000 revolutions. Thus a lower value correlates with a harder material. In the present invention, Einlehner hardness of the silica abrasive utilized in the present invention is determined by utilizing a brass screen. 100 g of silica is added to 1 L of water, and the slurry is rotated for 100,000 or 174,000 revolutions.

Other silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention.

*Foaming agents:* The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. Where present, the amount of of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants:* The compositions useful in the invention may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH3(CH2)mCH2(OCH2CH2)nOSO3X, wherein m is 6-16, e.g., 10, n is 1-6, e.g., 2, 3 or 4, and X is Na or K, for example sodium laureth-2 sulfate (CH3(CH2)10CH2(OCH2CH2)2OSO3Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C6-30 alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the composition of the invention, e.g., Composition 1, et seq., comprises sodium lauryl sulfate.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Flavoring Agents:* The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight e.g. about 0.5 to about 1.5% by weight.

*Polymers*: The oral care compositions of the invention may also include additional polymers to adjust the viscosity of the formulation or enhance the solubility of other ingredients. Such additional polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of about 0.5% to about 5.0% by weight of the total composition are used.

The compositions of the invention may include an anionic polymer, for example in an amount of from about 0.05 to about 5%. Such agents are known generally for use in dentifrice, although not for this particular application, useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 Sikes et al.

*Water:* Relatively high levels of water are present in the oral compositions of the invention. Water employed in the preparation of commercial oral compositions should be deionized and free of organic impurities. The amount of water in the compositions includes the free water which is added plus that amount which is introduced with other materials.

*Humectants:* Within certain embodiments of the oral compositions, it is also desirable to incorporate a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In one embodiment of the invention, the principal humectant is glycerin, which may be present at levels of greater than 25%, e.g. 25-35% about 30%, with 5% or less of other humectants.

*Other optional ingredients:* In addition to the above-described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Embodiments of the present invention are further described in the following examples. The examples are merely illustrative and do not in any way limit the scope of the invention as described and claimed.

### EXAMPLES

### Example 1 - Test formulations

A toothpaste base is formulated with about 29% glycerin and 37% water, with a thickening system comprised of natural cellulose (carrageenan), carboxymethyl cellulose (CMC) and thickening silica. Study prototypes are prepared by adding 3 levels of Avicel CL611 to the control formulation: 0.5%, 0.7% and 1% (Avicel CL611 is a mixture of 80-90% microcrystalline cellulose and 10-20% sodium carboxymethyl cellulose, available from FMC Corp.). The specific formulations are as follows:

| **Component** | **Current (%)** | **B (%)** | **C (%)** |
|---|---|---|---|
| Glycerin | 29.7 | 29.7 | 29.7 |
| Water | 38.83 | 38.13 | 37.83 |
| PVM/MA copolymer | 2 | 2 | 2 |
| Sodium hydroxide | 0.6 | 0.6 | 0.6 |
| Sodium fluoride | 0.32 | 0.32 | 0.32 |
| Sodium saccharin | 0.3 | 0.3 | 0.3 |
| Triclosan | 0.3 | 0.3 | 0.3 |
| Sodium lauryl sulfate | 1.58 | 1.58 | 1.58 |
| Carboxymethylcellulose | 1.1 | 1.1 | 1.1 |
| Carrageenan | 0.5 | 0.5 | 0.5 |
| Silica - abrasive | 20 | 20 | 20 |
| Silica - thickener | 3 | 3 | 3 |
| Titanium dioxide | 0.5 | 0.5 | 0.5 |
| Flavor | 1.27 | 1.27 | 1.27 |
| Avicel® CL-611 (about 81.2% - 88.7% microcrystalline cellulose and about 11.3-18.8% sodium carboxymethylcellulose) | 0.0 | 0.7 | 1.0 |
| Total | 100 | 100 | 100 |

The silica abrasive is a 1:3 mixture of a small particle silica (AC43 silica) and high cleaning silica (Sylodent VP5). The flavor delivery is evaluated by two independent experts with similar ratings in comparison to a low water commercial formulation having the same flavor, with results as follows:

**Organoleptic evaluation: flavor delivery from dentifrice formulation**

| | A | B | C |
|---|---|---|---|
| Expert 1 | not acceptable | acceptable | acceptable/most preferred |
| Expert 2 | not acceptable | acceptable | acceptable/most preferred |
| Viscosity (bku) | 43 | 44 | 48 |

0.5% Avicel does not provide any taste improvement, 0.7% is better than control and 1% has a significant improvement on flavor delivery. The addition of Avicel moreover results in higher viscosity compared to control, as well as higher elasticity, resulting in improved squeezing quality.

Prior formulations as disclosed in US Patent 5,601,803 involved low water formulations with very different humectant systems, e.g., 12% water with sorbitol, glycerin and polyethylene glycol vs the present 39% water with glycerin and copolymer. The gum dispersion process described in US Patent 5,601,803 is carried out in sorbitol, or water/glycerin mixture. It is found to be much better in the higher water systems to disperse the Avicel solely in water, prior to adding other ingredients. 0.7% appears to be the minimum level of Avicel CL611 needed to improve flavor delivery in the backbone studied and 1% is the optimal level to have the flavor profile comparable to a low water formula with the same level of flavor.

The high water formulation of formulation C therefore offers significant cost savings over a formulation with less water and correspondingly higher solids, yet provides good performance, taste and rheology.

### Example 2 - Consumer testing

In order to confirm the performance and consumer acceptability, consumer tests are carried out, comparing formulations B and C to a similar commercial formulation with the same flavoring, but with less water, higher solid content, including more abrasive, and no microcrystalline cellulose. The in-home placement test contained two cells: testing formulations B and C (new formulations) vs. current formulation. All respondents test 2 products (current and new), for a period of 10 days each (20 days in total) in place of their usual toothpaste. An equal number of respondents test each product 1st (50% will test the current product first and 50% will try a new formula first).

The formulations are as follows:

| **Component** | **Current (%)** | **B (%)** | **C (%)** |
|---|---|---|---|
| Glycerin | 16 | 29.7 | 29.7 |
| Water | 31.8 | 38.13 | 37.83 |
| PVM/MA copolymer | 2 | 2 | 2 |
| Sodium hydroxide | 0.6 | 0.6 | 0.6 |
| Sodium fluoride | 0.32 | 0.32 | 0.32 |
| Sodium saccharin | 0.3 | 0.3 | 0.3 |
| Triclosan | 0.3 | 0.3 | 0.3 |
| Sodium lauryl sulfate | 1.58 | 1.58 | 1.58 |
| Sodium carboxymethylcellulose | 0.6 | 1.1 | 1.1 |
| Carrageenan | 0.2 | 0.5 | 0.5 |
| Silica - abrasive | 34 | 20 | 20 |
| Silica - thickener | 0.0 | 3 | 3 |
| Titanium dioxide | 1.0 | 0.5 | 0.5 |
| Flavor | 1.27 | 1.27 | 1.27 |
| Avicel® CL-611 (about 81.2% - 88.7% microcrystalline cellulose and about 11.3-18.8% sodium carboxymethylcellulose) | 0.0 | 0.7 | 1.0 |
| Sorbitol | 10 | 0 | 0 |
| Total | 100 | 100 | 100 |

Stage 1 - Recruitment & placement: Recruitment from online panel of respondents, with interviews conducted online. Respondent is posted Current and New clearly labeled 'try 1st' and 'try 2nd'
Stage 2 - Reminder to switch to second product: After 10 days trial of the 1 st product, respondent is re-contacted to remind them to switch over to the second product.
Stage 3 - Final online recall: The respondent completes a final online questionnaire to collect preferences for the 2 products tried.
The recall questionnaire consists of around 20 questions of which one is an open ended question.

### Sample composition:

Sample size n=200 (at the recall stage in each of the 2 cells)

### Sample structure:

Men and women
   Gender: 70% Female, 30% Male
Aged 18-65
   18-34 = 50%; 35-65= 50%
   Loose quotas on Social grade - representative (50% ABC1 / 50% C2DE)
Responsible for buying toothpaste for themselves (either directly or at least choosing their brand)
None to have taken part in oral care research in the last 6 months
All to brush their teeth at least twice a day
All to visit the dentist at least once a year
People with self-reported allergies to personal care products and women who are pregnant or nursing will be excluded.

### Areas of Inquiry

- overall preference and reasons
- preference on attributes
- directional preference ratings

The new formulation with 1% Avicel is at least at parity to current commercial formulation on overall preference, while the 0.7% formulation falls slightly short, confirming initial assessment by experts. Both new formulae are at least at parity to current formulation on "has a taste you like" and on "dentist clean feeling that lasts" but on this last attribute, the new formula with 1% Avicel wins significantly vs. current on total sample whereas the new formula with 0.7% Avicel is at parity to current on total sample and fails vs. current with a loss among last 3 months users of current formulation.

New formula with 1% Avicel is superior to 0.7% formulation and has no weakness vs. current on image attributes e.g on freshness and cleanliness attributes with significant wins vs. current on "dentist clean feeling that lasts" as seen above and "leaves your teeth feeling really clean". It also wins significantly vs. current on consistency, texture, amount of foam. It is at parity vs. current on strength of flavour during brushing, strength of flavour after brushing, length flavour lasted, sweetness, mintiness.

## Claims

1. A dentifrice composition comprising by weight
a. 35-45% of water;
b. 0.7% to 1.2% of a component for enhancing flavor delivery and rheological profile, wherein the component comprises a blend of:
b1. 80% to 90% of a microcrystalline cellulose; and
b2. 10% to 20% of sodium carboxymethylcellulose;
wherein the weight percentage of b1. and b2. is based on the total weight of component; and
c. an effective amount of a silica abrasive, wherein the silica abrasive component comprises 15-25% of the composition,
wherein the composition is obtained or obtainable by premixing the blend of microcrystalline cellulose and sodium carboxymethyl cellulose in water prior to combination with the other ingredients.

2. The composition of claim 1 further comprising a synthetic anionic polymeric polycarboxylate.

3. The composition of claim 2 wherein the anionic polymer is a methyl vinyl ether/maleic anhydride (PVM/MA) copolymer having an average molecular weight (M.W.) of about 30,000 to about 1,000,000 and comprises 1-5% of the weight of the composition.

4. The composition according to any of claims 1-3 wherein the silica abrasive component comprises (a) a first population of silica abrasive particles, having a d50 of less than 5 microns, and (b) a second population of silica abrasive particles, having a d50 of greater than 8 microns, wherein the ratio of the first population to the second population by weight is between 1:2 and 1:4.

5. The composition according to any of the foregoing claims further comprising an effective amount of a fluoride ion source.

6. The composition according to any of the foregoing claims further comprising 25-35% of a humectant, and/or wherein the composition further comprises 1-2% sodium lauryl sulfate (SLS).

7. The composition according to any of the foregoing claims further comprising a viscosity modifying amount of one or more of xanthan gum, carrageenan, silica thickener, or combinations thereof.

8. The composition according to any of the foregoing claims further comprising an antibacterially effective amount of a natural extract antibacterial, non-ionic bacterial agent, triclosan magnolol, tetrahydromagnolol, butyl magnolol, honokiol, tetrahydrohonokiol or mixtures thereof.

9. The composition according to claim 1 further comprising
a) 25-35% glycerin as a humectant
b) 1-5% PVM/MA copolymer having an average molecular weight (M.W) of 30,000 to 1,000,000
c) 0.01-3% antibacterial agent.

10. The composition according to claim 1 further comprising
a) 25-35% glycerin as a humectant
b) 1-3% PVM/MA copolymer
c) 0.1-0.5% triclosan.

11. A method of making a dentifrice composition according to any of claims 1-10 comprising dispersing a blend of 80-90% microcrystalline cellulose and 10-20% sodium carboxymethyl cellulose in water, then admixing the remaining ingredients.

12. A dentifrice composition of any one of claims 1-10 for use in
(i) reducing plaque accumulation,
(ii) reducing or inhibiting demineralization and promote remineralization of the teeth,
(iii) inhibiting microbial biofilm formation in the oral cavity,
(iv) reducing or inhibiting gingivitis,
(v) reducing or inhibiting formation of dental caries,
(vi), reducing, repairing or inhibiting pre-carious lesions of the enamel,
(vii) cleaning the teeth and oral cavity,
(viii) reducing erosion,
(ix) whitening teeth; and/or
(x) promoting systemic health.

13. Use of microcrystalline cellulose in the manufacture of a dentifrice according to claim 1.

## Patentansprüche

1. Zahnreinigungsmittelzusammensetzung, umfassend bezogen auf das Gewicht
a. 35-45% Wasser;
b. 0,7% bis 1,2% eines Bestandteils zum Steigern von Geschmacksabgabe und rheologischem Profil, wobei der Bestandteil eine Mischung umfasst aus:
b1. 80% bis 90% mikrokristalliner Cellulose; und
b2. 10% bis 20% Natriumcarboxymethylcellulose;
wobei die Gewichtsprozentzahl von b1. und b2. auf dem Gesamtgewicht des Bestandteils basiert; und
c. eine wirksame Menge eines Silica-Abrasivstoffs, wobei der Silica-Abrasivstoffbestandteil 15-25% der Zusammensetzung umfasst,
wobei die Zusammensetzung durch Vormischen der Mischung aus mikrokristalliner Cellulose und Natriumcarboxymethylcellulose in Wasser vor dem Kombinieren mit den anderen Zutaten erhalten wird oder erhältlich ist.

2. Zusammensetzung nach Anspruch 1, weiterhin umfassend ein synthetisches anionisches polymeres Polycarboxylat.

3. Zusammensetzung nach Anspruch 2, wobei das anionische Polymer ein Methylvinylether/Maleinsäureanhydrid (PVM/MA)-Copolymer mit einem durchschnittlichen Molekulargewicht (M.W.) von etwa 30.000 bis etwa 1.000.000 ist und 1-5% des Gewichts der Zusammensetzung umfasst.

4. Zusammensetzung nach einem beliebigen der Ansprüche 1-3, wobei der Silica-Abrasivstoffbestandteil (a) eine erste Population an Silica-Abrasivstoffpartikeln mit einem d50 von weniger als 5 Mikrometern, und (b) eine zweite Population von Silica-Abrasivstoffpartikeln mit einem d50 von größer als 8 Mikrometern umfasst, wobei das Verhältnis der ersten Population zur zweiten Population bezogen auf das Gewicht zwischen 1:2 und 1:4 beträgt.

5. Zusammensetzung nach einem beliebigen der voranstehenden Ansprüche, weiterhin umfassend eine wirksame Menge einer Fluoridionenquelle.

6. Zusammensetzung nach einem beliebigen der voranstehenden Ansprüche, weiterhin umfassend 25-35% eines Feuchthaltemittels, und/oder wobei die Zusammensetzung weiterhin 1-2% Natriumlaurylsulfat (SLS) umfasst.

7. Zusammensetzung nach einem beliebigen der voranstehenden Ansprüche, weiterhin umfassend eine die Viskosität modifizierende Menge von einem oder mehreren von Xanthangummi, Carrageen, Silica-Verdickungsmittel oder Kombinationen davon.

8. Zusammensetzung nach einem beliebigen der voranstehenden Ansprüche, weiterhin umfassend eine antibakteriell wirksame Menge eines antibakteriellen natürlichen Extrakts, eines nicht ionischen bakteriellen Mittels, Triclosan, Magnolol, Tetrahydromagnolol, Butylmagnolol, Honokiol, Tetrahydrohonokiol oder Gemischen davon.

9. Zusammensetzung nach Anspruch 1, weiterhin umfassend
a) 25-35% Glycerin als ein Feuchthaltemittel
b) 1-5% PVM/MA-Copolymer mit einem durchschnittlichen Molekulargewicht (M.W.) von 30.000 bis 1.000.000
c) 0,01-3% antibakterielles Mittel.

10. Zusammensetzung nach Anspruch 1, weiterhin umfassend
a) 25-35% Glycerin als ein Feuchthaltemittel
b) 1-3% PVM/MA-Copolymer
c) 0,1-0,5% Triclosan.

11. Verfahren zum Herstellen einer Zahnreinigungsmittelzusammensetzung gemäß einem beliebigen der Ansprüche 1-10, umfassend Dispergieren einer Mischung von 80-90% mikrokristalliner Cellulose und 10-20% Natriumcarboxymethylcellulose in Wasser, dann Beimischen der verbleibenden Zutaten.

12. Zahnreinigungsmittelzusammensetzung nach einem beliebigen der Ansprüche 1-10 zur Verwendung beim
(i) Verringern von Plaque-Ansammlung,
(ii) Verringern oder Inhibieren von Demineralisierung und Fördern von Remineralisierung der Zähne,
(iii) Inhibieren von mikrobieller Biofilmbildung in der Mundhöhle,
(iv) Verringern oder Inhibieren von Gingivitis,
(v) Verringern oder Inhibieren der Bildung von Zahnkaries,
(vi) Verringern, Reparieren oder Inhibieren von Präkaries-Zahnschmelzläsionen,
(vii) Reinigen der Zähne und Mundhöhle,
(viii) Verringern von Erosion,
(ix) Aufhellen von Zähnen; und/oder
(x) Fördern der systemischen Gesundheit.

13. Verwendung von mikrokristalliner Cellulose beim Herstellen eines Zahnreinigungsmittels gemäß Anspruch 1.

## Revendications

1. Composition de dentifrice comprenant en poids
a. 35 à 45 % d'eau ;
b. 0,7 % à 1,2 % d'un constituant pour améliorer l'administration d'arôme et le profil rhéologique, dans lequel le constituant comprend un mélange de :
b1. 80 % à 90 % d'une cellulose microcristalline ; et
b2. 10 % à 20 % de carboxyméthylcellulose sodique ;
dans lequel le pourcentage en poids de b1. et b2. est fondé sur le poids total du constituant ; et
c. une quantité efficace d'un abrasif à base de silice, dans lequel le constituant abrasif à base de silice constitue 15 à 25 % de la composition,
dans laquelle la composition est obtenue ou peut être obtenue par pré-mélange du mélange de cellulose microcristalline et de carboxyméthylcellulose sodique dans de l'eau avant la combinaison avec les autres ingrédients.

2. Composition selon la revendication 1, comprenant en outre un polycarboxylate polymère anionique synthétique.

3. Composition selon la revendication 2, dans laquelle le polymère anionique est un copolymère d'éther méthylique de vinyle/anhydride maléique (PVM/MA) ayant un poids moléculaire moyen (MW) d'environ 30 000 à environ 1 000 000 et constitue 1 à 5 % du poids du composition.

4. Composition selon l'une quelconque des revendications 1 à 3, dans laquelle le constituant abrasif à base de silice comprend (a) une première population de particules de silice abrasives, ayant un d50 inférieur à 5 microns, et (b) une deuxième population de particules de silice abrasives, ayant un d50 supérieur à 8 microns, dans laquelle le rapport de la première population à la deuxième population en poids est compris entre 1:2 et 1:4.

5. Composition selon l'une quelconque des revendications précédentes, comprenant en outre une quantité efficace d'une source d'ion fluorure.

6. Composition selon l'une quelconque des revendications précédentes, comprenant en outre 25 à 35 % d'un humectant, et/ou dans laquelle la composition comprend en outre 1 à 2 % de laurylsulfate de sodium (SLS).

7. Composition selon l'une quelconque des revendications précédentes, comprenant en outre une quantité modifiant la viscosité d'un ou de plusieurs éléments parmi la gomme de xanthane, le carraghénane, un épaississant de silice ou les combinaisons de ceux-ci.

8. Composition selon l'une quelconque des revendications précédentes, comprenant en outre une quantité efficace au plan antibactérien d'un extrait naturel antibactérien, d'un agent bactérien non ionique, de triclosan magnolol, de tétrahydromagnolol, de butyl magnolol, d'honokiol, de tétrahydrohonokiol ou de mélanges de ceux-ci.

9. Composition selon la revendication 1, comprenant en outre
a) 25 à 35 % de glycérine en tant qu'humectant
b) 1 à 5 % de copolymère de PVM/MA ayant un poids moléculaire moyen (MW) de 30 000 à 1 000 000
c) 0,01 à 3 % d'agent antibactérien.

10. Composition selon la revendication 1, comprenant en outre
a) 25 à 35 % de glycérine en tant qu'humectant
b) 1 à 3 % de copolymère de PVM/MA
c) 0,1 à 0,5 % de triclosan.

11. Procédé de fabrication d'une composition de dentifrice selon l'une quelconque des revendications 1 à 10, comprenant la dispersion d'un mélange de 80 à 90 % de cellulose microcristalline et de 10 à 20 % de carboxyméthylcellulose sodique dans de l'eau, puis le mélange des ingrédients restants.

12. Composition de dentifrice selon l'une quelconque des revendications 1 à 10, pour une utilisation dans
(i) la réduction de l'accumulation de plaque,
(ii) la réduction ou l'inhibition de la déminéralisation et la promotion de la reminéralisation des dents,
(iii) l'inhibition de la formation de biofilm microbien dans la cavité buccale,
(iv) la réduction ou l'inhibition de la gingivite,
(v) la réduction ou l'inhibition de la formation des caries dentaires,
(vi) la réduction, la réparation ou l'inhibition des lésions pré-carieuses de l'émail,
(vii) le nettoyage des dents et de la cavité buccale,
(viii) la réduction de l'érosion,
(ix) le blanchiment des dents ; et/ou
(x) la promotion de la santé systémique.

13. Utilisation de cellulose microcristalline dans la fabrication d'un dentifrice selon la revendication 1.
